(19) Europäisches Patentamt
European Patent Office
Office européen des brevets

(11) Numéro de publication: **0 297 944 B1**

# (12) FASCICULE DE BREVET EUROPEEN

(45) Date de publication de fascicule du brevet: **15.12.93**

(51) Int. Cl.[5]: **C12N 9/24**, //(C12N9/24, C12R1:66)

(21) Numéro de dépôt: **88401501.7**

(22) Date de dépôt: **16.06.88**

Le dossier contient des informations techniques présentées postérieurement au dépôt de la demande et ne figurant pas dans le présent fascicule.

## (54) Production d'une enzyme du type beta-glucuronidase, hydrolyse de la glycyrrhizine et production d'acide 18 beta-glycyrrhétinique.

(30) Priorité: **16.06.87 FR 8708384**

(43) Date de publication de la demande: **04.01.89 Bulletin 89/01**

(45) Mention de la délivrance du brevet: **15.12.93 Bulletin 93/50**

(84) Etats contractants désignés: **AT BE CH DE ES FR GB GR IT LI LU NL SE**

(56) Documents cités:
**US-A- 3 801 461**

**CHEMICAL ABSTRACTS, vol. 105, 1986, page 608, résumé no. 41249t, Columbus,Ohio, US**

**AGRICULTURAL BIOLOGICAL CHEMISTRY, vol. 50, no. 3, 1986, pages 687-692, JP; T.MURO et al.: "Purification and some properties of glycyrrhizinic acid hydrolase from Aspergillus niger GRM3"**

**CHEMICAL ABSTRACTS, vol. 98, no. 25, 20 juin 1983, page 381, résumé no.221800m, Columbus, Ohio, US**

(73) Titulaire: **PERNOD-RICARD**
**142, Boulevard Haussmann**
**F-75008 Paris(FR)**

(72) Inventeur: **Ballester, Maryvonne**
**19, rue Bravet**
**F-13005 Marseille(FR)**
Inventeur: **Giallo, Jacqueline**
**6, Place Charles Bichi**
**F-13015 Marseille(FR)**
Inventeur: **Zicavo, Pascaline**
**24, rue du Petit Chantier**
**F-13007 Marseille(FR)**
Inventeur: **Ballester, Jean-Michel**
**19, rue Bravet**
**F-13005 Marseille(FR)**
Inventeur: **Bricout, Jacques**
**Evry/Gregy/Yerres**
**F-77166 Grisy Suisnes(FR)**

(74) Mandataire: **Warcoin, Jacques et al**
**Cabinet Régimbeau**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 297 944 B1

## Description

La présente invention concerne un procédé de production d'une enzyme fongique et son utilisation à l'hydrolyse de la glycyrrhizine pour obtenir des extraits de réglisse à teneur réduite en glycyrrhizine ou pour obtenir de l'acide 18 β-glycyrrhétinique.

On sait en effet que la glycyrrhizine est à l'origine de problèmes cardio-vasculaires, notamment accroissement de la tension artérielle chez des sujets sensibles. Pour certains usages, il est souhaitable de réduire la teneur en glycyrrhizine ou même de supprimer sa présence dans les extraits de réglisse.

Par ailleurs, l'acide 18 β-glycyrrhétinique est un médicament reconnu qui intervient dans de nombreuses thérapeutiques.

Plus précisément, la présente invention concerne les enzymes β-glucuronidases.

On sait que les β-glucuronidases sont des enzymes capables d'hydrolyser des β-glucuronides et de réaliser le transfert des radicaux glucuronyls sur les alcools, selon le schéma :

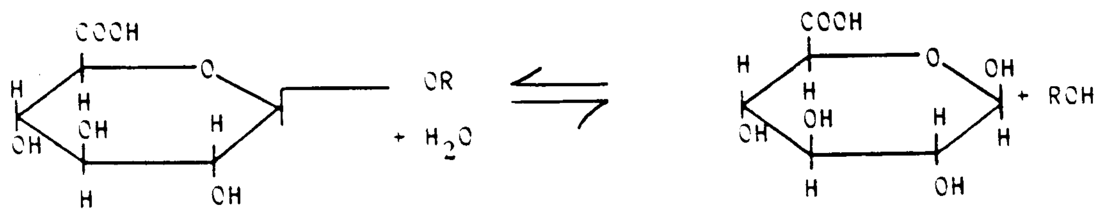

Or la glycyrrhizine est un éther diglucuronique de l'acide 18 β glycyrrhétinique et donc hydrolysable par de telles enzymes avec production d'acide 18 β glycyrrhétinique.

De nombreux auteurs ont déjà décrit plusieurs β-glucuronidases issues de sources diverses : foie de souris, glande préputiale de la femelle du rat, embryon de Xenopus laevis, Locusta migratoria, Helix pomatia, Patella vulgata, Scutellaria baicalensis.

Plusieurs glucuronidases de micro-organismes sont connues comme celles d'Escherichia coli, de streptocoques, d'Aeromonas hydrophila, Aeromonas punctata, Aeromonas salmonicide.

Le but de la présente invention est d'obtenir une β-glucuronidase microbienne très active, notamment vis-à-vis de l'hydrolyse de la glycyrrhizine.

Pour ce faire, à partir d'échantillons d'eaux, de sols, de denrées alimentaires, on a isolé plusieurs micro-organismes se développant en synthétisant une β-glucuronidase active.

La présente invention a en effet pour objet un procédé de production d'une enzyme du type β-glucuronidase comportant la culture d'un micro-organisme, effectuée dans un milieu comportant au moins une source de carbone et une source d'azote, et l'extraction et la purification de l'enzyme à partir de la biomasse et/ou du milieu de culture, caractérisé en ce que l'enzyme est produite par la culture d'un micro-organisme du type Aspergillus ou de ses variants et mutants dans un milieu de culture contenant comme source de carbone une substance comportant un ou plusieurs glucuronides.

Parmi ces micro-organismes on peut citer avantageusement les Aspergillus terreus et Aspergillus niger dont on a découvert qu'ils avaient selon l'invention la propriété de synthétiser une β-glucuronidase très active et très stable dans l'alcool et à la chaleur.

Notamment parmi ces micro-organismes producteurs, on peut citer la moisissure, dont les caractéristiques morphologiques et biochimiques montrent qu'il s'agit d'Aspergillus terreus. Cette moisissure a été isolée à partir de denrées alimentaires. Ses caractéristiques microbiologiques sont les suivantes :

. production de conidies chamois à brunes, avec pigmentation jaune ocre de la gélose,
. têtes conidiennes biseriées, hémisphériques ; les métules ne couvrant que la moitié supérieure de la vésicule avec présence de conidies globuleuses.

Les caractéristiques de la moisissure d'Aspergillus terreus ne sont pas limitées à ce qui est mentionné ci-dessus, cette moisissure étant susceptible d'être modifiée artificiellement ou naturellement. Mais, pourvu qu'elle possède au moins la capacité de production de la β-glucuronidase, il va sans dire que les variantes et mutantes de cette moisissure peuvent aussi être utilisées dans le procédé de l'invention.

Par conséquent, il est entendu que parmi les espèces de moisissure avantageuses utilisées dans la présente invention, il faut englober en plus des moisissures Aspergillus terreus et Aspergillus niger, les variantes et mutantes qui possèdent la plupart des propriétés distinctives des deux moisissures génératrices.

Les cultures d'Aspergillus sont effectuées sur un milieu convenable contenant au moins une source de carbone , une source d'azote et une substance inorganique de préférence.

La source de carbone peut être de préférence la glycyrrhizine, par exemple sous forme d'un extrait aqueux de réglisse.

La source d'azote est constituée avantageusement d'un sel d'ammonium, de sels de nitrate, d'acides aminés ou de toutes autres sources d'azote conventionnelles.

Le mono-ammonium de glycyrrhizinate peut être utilisé comme seule source de carbone et d'azote.

La croissance est meilleure quand on ajoute au milieu de culture des sels minéraux, tels que du sulfate de magnésium, du chlorure de calcium, du sulfate de fer, du sulfate de cuivre, du molybdate de sodium, du chlorure de calcium, du sulfate de manganèse, ou tous sels inorganiques pouvant favoriser la croissance des moisissures.

De même, l'addition de vitamines telles que l'acide folique, la biotine , la choline, la nicotinamide, le panthothénate de calcium, le pyridoxal, la thiamine, la riboflavine, ou toute autre vitamine, favorise la croissance des moisissures Aspergillus, notamment Aspergillus terreus et Aspergillus niger produisant la $\beta$-glucuronidase.

Bien entendu, les proportions des sources de carbone, d'azote et des sels inorganiques, ainsi que la composition du milieu lui-même peuvent varier suivant les facultés d'adaptation de la moisissure qui est utilisée pour la production de $\beta$-glucuronidase.

Comme température optimale pour le développement de ces micro-organismes, on peut utiliser normalement une température comprise entre 28 °C et 45 °C et de préférence entre 37 °C et 44 °C.

Le pH du milieu particulièrement approprié peut être compris entre 6 et 8.

La culture est effectuée pendant une période de dix jours.

Bien que la culture puisse être effectuée en milieu solide ou liquide, on obtient de meilleurs résultats en milieu liquide.

De préférence, la culture est réalisée dans des conditions convenant aux micro-organismes aérobies.

Au laboratoire, les meilleurs résultats sont obtenus en utilisant 500 ml de milieu de culture dans un erlen de deux litres, ensemencés avec des spores d'Aspergillus terreus ou des spores d'Aspergillus niger.

Les meilleurs résultats sont obtenus en utilisant 500 ml de milieu de culture dans un erlen de deux litres, ensemencés avec des spores d'Aspergillus terreus ou des spores d'Aspergillus niger.

Pendant les premiers jours de la croissance, l'activité $\beta$-glucuronidasique est localisée dans les cellules, aussi bien sur un milieu liquide, que sur un milieu solide. Ensuite, la $\beta$-glucuronidase est excrétée dans le milieu de culture.

L'invention à également pour objet un procédé comportant l'extraction de l'enzyme à partir du mycélium Aspergillus obtenu en milieu liquide ou solide, ou à partir de la phase surnageante des cultures et sa purification, caractérise en ce que lorsque la solution enzymatique extraite contient de la $\beta$-glucuronidase complexée avec de l'acide 18 $\beta$-glycyrrhétinique, ladite solution est additionnée d'urée dissociant le complexe, puis les deux produits sont séparés après élimination de l'urée.

En effet, on peut préparer la $\beta$-glucuronidase, à partir du mycélium d'Aspergillus, par exemple d'Aspergillus terreus obtenu en milieu liquide ou en milieu solide, ou à partir de la phase surnageante des cultures liquides.

Pour obtenir la $\beta$-glucuronidase à partir du mycélium, on réalise une séparation par centrifugation du mycélium et du milieu de culture liquide. Le mycélium est cassé par exemple dans un broyeur à bille comme du type Bead Beater. La $\beta$-glucuronidase est séparée des débris cellulaires par centrifugation.

Dans le cas où la moisissure est cultivée sur un milieu solide, le mycélium est récupéré en grattant la surface du milieu de culture. Le mycélium est cassé et l'enzyme est séparée des débris cellulaires comme précédemment. Toutes ces premières étapes de séparation du mycélium et de l'enzyme peuvent se dérouler à température ambiante ou à 4 °C.

Pour obtenir la $\beta$-glucuronidase par exemple d'Aspergillus terreus à partir d'une culture liquide âgée, donc une culture qui possède une activité $\beta$-glucuronidasique dans le mycélium et dans le milieu de culture, on peut procéder de la manière suivante : le volume de culture est filtré sur étamine, cette filtration permet de séparer le surnageant et le mycélium, qui sont ensuite traités séparément. On lave le mycélium avec de l'eau osmosée et on filtre à nouveau la suspension mycélienne ainsi obtenue sur étamine. Le mycélium lavé est récolté, puis cassé par exemple dans un broyeur à billes. La $\beta$-glucuronidase est ensuite séparée des débris cellulaires par centrifugation à 15 000 g à 4 °C pendant trente minutes. Après centrifugation, on précipite les substances contenues dans le surnageant en ajoutant du chlorure de sodium (0,285 M). Le précipité est séparé de la phase liquide par centrifugation. Le culot contient des molécules biologiques, de la glycyrrhizine et de l'acide glycyrrhétinique. La $\beta$-glucuronidase se trouve dans le surnageant . Sur ce surnageant, on opère une ultrafiltration à l'aide d'un support dont le pouvoir de coupure moyen permet de retenir toutes les molécules dont le poids moléculaire moyen est supérieur à 10 000 daltons (la concentration peut être réalisée avec des supports de filtration ayant un pouvoir de coupure

inférieur ou égal à 10 000 daltons). La solution enzymatique concentrée contenant la β-glucuronidase est alors envoyée sur une colonne d'échange d'ions type diéthylaminoéthyl pouvant être chargée avec des groupes fonctionnels divers, ou sur une autre matrice possédant les mêmes propriétés. La colonne est équilibrée à pH 7, la chromatographie d'échange d'ions peut être indifféremment réalisée à la température ambiante ou à 4°C. Après filtration de la β-glucuronidase, on applique sur la colonne un gradient croissant de chlorure de sodium allant de 0 a 0,75 M en chlorure de sodium. Le sel est mélangé, avant son application, au tampon qui a servi à équilibrer la colonne. On récupère la β-glucuronidase dans le tampon de sortie, quand la molarité en chlorure de sodium à l'intérieur de la colonne de chromatographie atteint 0,2 M en chlorure de sodium.

La β-glucuronidase ainsi partiellement purifiée est concentrée par ultrafiltration sur une membrane dont la porosité permet d'exclure des molécules dont le poids moyen est inférieur à 10 000 daltons.

Une chromatographie d'exclusion sur un gel, tel que les Séphadex G75, G100, G150, G200, les ultragels ACA 44, ACA 34 et ACA 22, et tous les gels qui permettent de séparer les molécules en fonction de leur poids moléculaire, est ensuite utilisée pour terminer la purification de la β-glucuronidase d'Aspergillus terreus.

Le traitement du surnageant obtenu après filtration sur étamine est identique à celui du milieu de culture débarrassé du mycélium d'Aspergillus terreus. Il comprend les étapes suivantes : le surnageant ou le milieu de culture acellulaire est ultrafiltré à l'aide d'une membrane dont le pouvoir de coupure moyen permet de retenir la β-glucuronidase. La solution enzymatique concentrée contenant la β-glucuronidase est alors envoyée sur une colonne d'échange d'ions, type diéthylaminoéthyl, pouvant être chargée avec des groupes fonctionnels divers, ou sur une autre matrice possédant les mêmes propriétés. Le protocole de purification est ensuite identique à celui adopté pour purifier la β-glucuronidase à partir de l'extrait cellulaire contenant le mycélium broyé.

Après l'ultrafiltration, la solution enzymatique contenant la β-glucuronidase peut contenir de l'acide 18 β-glycyrrhétinique en quantité suffisante pour troubler le milieu et gêner la purification. On ajoute alors dans la suspension de l'urée jusqu'à obtenir une concentration molaire en urée. La présence de l'urée permet de dissocier le complexe acide 18 β-glycyrrhétinique et β-glucuronidase facilitant ainsi la récupération des deux produits. On élimine l'urée en réalisant une chromatographie d'exclusion sur un gel de type Séphadex G10 ou G25, ou tout autre gel se prêtant bien aux opérations de tamisage moléculaire et possédant des domaines d'exclusion proches de ceux des Séphadex G10 ou G25.

La solution enzymatique contenant la β-glucuronidase est alors envoyée, comme on l'a décrit plus haut, sur une colonne d'échange d'ions de type diéthylaminoéthyl pouvant être chargée avec des groupes fonctionnels divers ou sur une autre matrice possédant les mêmes propriétés. Le protocole de purification est ensuite identique à celui adopté pour purifier la β-glucuronidase à partir de l'extrait cellulaire contenant le mycélium broyé.

On a mis au point une autre technique de séparation de l'acide 18 β-glycyrrhétinique et de la β-glucuronidase en précipitant l'activité β-glucuronidasique avec du sulfate d'ammonium à 80 % de saturation. Le précipité est séparé de la phase liquide par filtration sur étamine. On ajoute alors au précipité de l'urée jusqu'à obtenir une concentration molaire en urée. Après dissociation de la β-glucuronidase et de l'acide 18 β-glycyrrhétinique, on élimine l'urée en réalisant une chromatographie d'exclusion sur un gel de type Séphadex G10 ou G25, ou tout autre gel se prêtant bien aux opérations de tamisage moléculaire et possédant des domaines d'exclusion proches de ceux des Séphadex G10 ou G25.

La solution enzymatique contenant la β-glucuronidase est alors envoyée comme on l'a décrit plus haut sur une colonne d'échange d'ions de type diéthylaminoéthyl, pouvant être chargée avec des groupes fonctionnels divers, ou sur un autre matrice possédant les mêmes propriétés. Le protocole de purification est ensuite identique à celui adopté pour purifier la β-glucuronidase à partir de l'extrait cellulaire contenant le mycélium broyé.

La présente invention a également pour objet une nouvelle enzyme de type β-glucuronidase, caractérisée en ce qu'elle est obtenue par le procédé selon l'invention.

Ces enzymes β-glucuronidases sont caractérisées par un maximum d'activité entre 40 et 50°C, de préférence 45°C et à pH entre 5 et 7, de préférence 6.

Notamment, la β-glucuronidase d'Aspergillus terreus, ainsi purifiée, pourvu qu'elle soit placée en phase aqueuse, possède un maximum d'activité à 45°C, confère 100 % de son activité entre 43 et 48°C et 83 % de son activité à 60°C vis-à-vis de la phénolphtaléine glucuronidase.

La β-glucuronidase d'Aspergillus terreus a un maximum d'activité à pH 6 quand elle se trouve en phase aqueuse. Elle conserve 80 % de son activité à pH 5 et à pH 7.

L'enzyme selon l'invention présente un poids moléculaire de l'ordre de 95.000 daltons.

D'autre part, cette enzyme peut être immobilisée sur un support insoluble comme le nylon activé au glutaraldéhyde ou sur billes acryliques activées à l'oxirane.

Cette β-glucuronidase exempte d'activité sulfatase a une meilleure affinité vis-à-vis de la glycyrrhizine que les enzymes actuellement disponibles.

La présente invention a donc également pour objet un procédé d'hydrolyse d'une solution de glycyrrhizine ou d'extraits végétaux en contenant et contenant ou non de l'alcool éthylique jusqu'à 20 % en volume, caractérisé en ce qu'on ajoute à la solution de la β-glucuronidase obtenue par le procédé selon la présent invention.

Il est à noter en effet que, de manière très intéressante, la présence d'éthanol n'inhibe pas l'hydrolyse de la glycyrrhizine par cette enzyme pour autant que la concentration en alcool reste inférieure à 20% en volume.

Sur le plan de l'utilisation, la possibilité d'employer l'enzyme dans un milieu contenant 20 % d'alcool est très intéressante car c'est une garantie pour la stérilité.

Avantageusement, on opère à pH entre 4,5 et 6,5, et de préférence 5,5.

De même, la température la plus appropriée sera comprise entre 35 et 55 ° C, de préférence 45 ° C.

Les enzymes β-glucuronidases obtenues à partir de cultures d'Aspergillus, notamment d'Aspergillus terreus selon les procédés décrits précédemment (enzymes intra- et extra-cellulaires sont donc particulièrement intéressantes vis-à-vis de leur action envers la glycyrrhizine).

Lorsque l'on suit l'hydrolyse de la glycyrrhizine en HPLC, sous l'action de l'enzyme, on constate une diminution de la teneur en glycyrrhizine et l'apparition d'acide 18 β-glycyrrhétinique, ainsi qu'un pic de polarité intermédiaire qui est le mon-glucuronide de l'acide 18 β-glycyrrhétinique. Lorsque la réaction est arrivée à complétion, ce pic intermédiaire est extrêmement faible et on récupère l'acide 18 β-glycyrrhétinique qui a ce pH précipite.

On obtient l'acide 18 β-glycyrrhétinique cristallisé pur, ce qui représente un avantage très important par rapport aux méthodes actuellement utilisées pour produire l'acide 18 β-glycyrrhétinique par hydrolyse chimique de la glycyrrhizine.

Quand on hydrolyse la glycyrrhizine en phase aqueuse avec la β-glucuronidase d'Aspergillus terreus, on obtient de l'acide 18 β-glycyrrhétinique avec un rendement pouvant aller jusqu'à 90 %.

La présente invention a donc également pour objet un procédé de production d'acide 18 β-glycyrrhétinique, caractérisé en ce qu'on hydrolyse la glycyrrhizine avec ou un extrait végétal en contenant selon le procédé d'hydrolyse de la glycyrrhizine de l'invention.

En effet, comme on l'a vu précédemment, l'enzyme isolée à partir d'Aspergillus terreus par exemple permet une hydrolyse complète de la glycyrrhizine et la récupération quantitative de l'acide 18 β-glycyrrhétinique qui précipite de la solution aqueuse ou qui peut être extrait par un solvant organique.

Enfin, la présente invention a pour objet des extraits de réglisse pouvant contenir ou non de l'alcool éthylique, caractérisés en ce qu'ils présentent une teneur réduite ou nulle en glycyrrhizine et dépourvue d'acide 18 β-glycyrrhétinique.

L'hydrolyse de la glycyrrhizine, selon le procédé de l'invention, a en outre l'avantage qu'elle conserve l'intégralité des autres consituants de la réglisse.

D'autres avantages et caractéristiques de la présente invention apparaîtront à la lumière des exemples qui suivent.

EXEMPLE I

Aspergillus terreus est ensemencé sur un milieu liquide dont la composition est la suivante :

| | |
|---|---|
| - mono-ammonium de glycyrrhizinate | 8 g/l |
| - tampon phosphate $KH_2PO_4$ | 1,36 g/l |
| - nitrate d'ammonium | 1 g/l |
| - solution a | 10 ml/l |
| - solution b | 10 ml/l |
| - le pH de ce milieu est fixé à 6,5 | |

La composition de la solution a est la suivante :

| | |
|---|---|
| - $MgSO_4$ | 6 g/l |
| - $CaCl_2$, $2H_2O$ | 0,015 g/l |
| - $FeSO_4$, $7H_2O$ | 0,028 g/l |
| - $ZnSO_4$, $7H_2O$ | 1,14 g/l |
| - $CuSO_4$, $4H_2O$ | 0,025 g/l |
| - $Na_2$ Mo $O_4$, $2H_2O$ | 0,024 g/l |
| - CaCl, $6H_2O$ | 0,024 g/l |
| - $MnSO_4$, $H_2O$ | 0,084 g/l |

La composition de la solution b est la suivante :

| | |
|---|---|
| - acide folique | 1 mg/l |
| - choline | 1 mg/l |
| - inositol | 1 mg/l |
| - nicotinamide | 1 mg/l |
| - panthothénate | 1 mg/l |
| - pyridoxal | 1 mg/l |
| - thiamine | 1 mg/l |
| - riboflavine | 0,01 mg/l |

Le mono-ammonium de glycyrrhizinate et la solution b sont stérilisés par filtration sur des membranes de porosité égale à 0,22 µM, avant d'être incorporés au milieu de culture qui, lui, est stérilisé par la chaleur à 120 °C.

A partir d'une culture d'un litre d'Aspergillus terreus âgée de quinze jours et ayant poussé à 37 °C, on a mis en évidence que 25 % de l'activité glucuronidase se trouve dans le surnageant et 75 % sous forme intracellulaire.

On réalise un dosage de l'activité β-glucuronidase à 37 °C pendant trente minutes, en ajoutant cent microlitres de la solution enzymatique dans 2 ml de tampon phosphate 20 mM à pH 7 contenant de la phénolphtaléine glucuronidate $1,92.10^{-4}$ M. Au bout de trente minutes, la réaction est arrêtée par addition de 3 ml de tampon glycine 0,024 M - NaOH 0,04 M à pH 10,5. La densité optique est lue à 540 nanomètres. L'unité β-glucuronidase est la quantité d'enzyme libérant 1 µg de phénolphtaléine par heure à pH 7.

On centrifuge le milieu de culture dans une centrifugeuse type BECKMAN réfrigérée à 4 °C, à 15 000 g pendant trente minutes. Le surnageant de centrifugation est traité séparément du culot mycélien.

On concentre le surnageant obtenu après centrifugation, qui est semblable à l'extrait que l'on peut réaliser en filtrant le milieu de culture sur étamine, sur une membrane type AMICON XM 10. Cette opération est renouvelée plusieurs fois à 4 °C, de façon à obtenir une fraction enzymatique à pH 7 et possédant une force ionique inférieure à la force ionique du tampon qui est ensuite employé pour la chromatographie d'échange d'ions.

La solution enzymatique ainsi préparée est envoyée par l'intermédiaire d'une pompe périlstatique au sommet d'une colonne contenant une matrice de diéthylaminoéthyl type DEAE Sépharose CL6B. On lave la matrice avec le tampon qui à été utilisé lors de la fixation de l'enzyme sur la matrice. On applique ensuite un gradient salin allant de 0 à 0,1 M en chlorure de sodium, sans changer le pH du tampon. On récupère l'enzyme dans le tampon de sortie quand la molarité en chlorure de sodium dans la colonne atteint 0,02 M.

On élimine le chlorure de sodium et on concentre la solution enzymatique issue de la chromatographie d'échange d'ions sur une membrane AMICON XM 10. La solution enzymatique concentrée est envoyée ensuite sur une colonne de chromatographie d'exclusion, chargée avec de l'Ultrogel ACA 34, la phase aqueuse étant constituée d'un tampon phosphate à pH 7 et de chlorure de sodium à 0,1 M. Après cette étape, la β-glucuronidase libre ou fixée peut être utilisée pour la scission enzymatique de la molécule de glycyrrhizine et l'obtention d'acide 18 β-glycyrrhétinique.

EXEMPLE 2

On récupère le surnageant d'une culture âgée de quinze jours, par filtration sur étamine. Puis on ajoute de l'urée en poudre jusqu'à obtenir une concentration en urée égale à 1 M.

Cette fraction est ensuite envoyée sur une colonne de tamisage moléculaire dont le domaine de fractionnement permet de séparer des protéines ayant un poids moléculaire compris entre 1 000 et 5 000 daltons.

En tête de colonne, on obtient l'activité β-glucuronidase, tandis que l'acide 18-β-glycyrrhétinique est récupéré en fin de chromatographie.

L'acide 18-β-glycyrrhétinique peut être purifié à l'aide d'une extraction au chloroforme ou bien d'une précipitation.

EXEMPLE III

L'enzyme a été comparée aux autres β-glucuronidases commerciales.

Pour chaque enzyme, on a utilisé l'activité ainsi définie : 1 unité enzymatique libère 1 mg de phénolphtaléine à partir de glucuronide de phénolphtaléine par heure à 37 °C au pH optimum de l'enzyme. Tous les essais ont été conduits pendant 16 h à 40 °C en utilisant 0,2 mg de glycyrrhizine et 40 unités enzymatiques. Pour chaque enzyme, le pH optimum d'activité vis-à-vis de la glycyrrhizine a été déterminé. Dans tous les cas un pH = 5 convenait très bien.

| ORIGINE DE L'ENZYME | ACIDE 18 β-GLYCYRRHETINIQUE FORME EN % |
|---|---|
| *Aspergillus terreus* | 67 |
| *Patella vulgata* | 59 |
| Abalone | 44 |
| Hélix Pomatia | 42 |

1) 5 mg de glycyrrhizine dans 3 ml d'eau, ajustés à pH 5,2 sont additionnés de 1.000 unités enzymatiques de β-glucuronidase d'Aspergillus terreus. Après 48 h à 40 °C, on récupère 2,4 mg d'acide 18 β-glycyrrhétinique.

2) 5 mg de glycyrrhizine sont dilués dans 2 ml d'eau, le pH est ajusté à 5,2 et on rajoute 0,5 ml d'éthanol et 500 unités enzymatiques. On laisse 48 h à 40 °C. On centrifuge le précipité formé, constitué de 18 β-glycyrrhétinique pur. On rajoute 2,5 mg de glycyrrhizine, puis on laisse à 40 °C/2 jours. On centrifuge à nouveau le précipité et on recommence la même opération trois fois de suite. On a ainsi pu hydrolyser 15 mg de glycyrrhizine avec 500 unités de β-glucuronidase en huit jours à 40 °C. Cette expérience montre la grande stabilité de cette enzyme.

3) 4 mg de glycyrrhizine sont dilués dans 4 ml d'eau, le pH est ajusté à 5,2 et on ajoute 1.000 unités d'enzyme et 2 ml d'acétate d'éthyle. On laisse sous agitation pendant 48 h à température ambiante. On récupère dans la phase acétate d'éthyle 1,75 mg d'acide 18 β-glycyrrhétinique pur, soit 85 % de la dose théorique.

4) A 5 ml d'un extrait de réglisse hydroalcoolique à 20 % en volume d'alcool ajusté à pH 5,3 et contenant 41 mg de glycyrrhizine, on ajoute 2.500 unités de β-glucuronidase. On laisse 48 h à 40 °C et on centrifuge à 0 °C. Le surnageant ne contient alors que 11 mg de glycyrrhizine, des traces de mon-glucuronide de l'acide 18 β-glycyrrhétinique, ainsi que l'intégralité des autres constituants de la réglisse, en particulier les chalcones qui confèrent la couleur aux extraits de réglisse. Ainsi donc l'enzyme extrait d'*Aspergillus terreus* permet de réduire fortement, voire complètement, la teneur en glycyrrhizine d'extraits de réglisse utilisés en Industrie Alimentaire, sans modifier les autres constituants de la plante.

EXEMPLE IV : FIXATION DE L'ENZYME

L'enzyme est fixé sur des billes acryliques activées à l'oxirane (Eupergit C de la Société Röhm Pharma).

L'enzyme est mis en solution dans un tampon $KH_2PO_4$ 1 M., l'activité de cette solution étant de 20 à 50.OOO unités/ml. 3,5 ml de la solution enzymatique sont mis en contact avec 1 g d'Eupergit C pendant 24 heures à température ambiante. Les billes sont ensuite lavées avec un tampon phosphate 0,1 M.

Elles peuvent être ensuite utilisées plusieurs fois de suite pour hydrolyser, soit une solution de glycyrrhizine, soit la glycyrrhizine contenue dans un extrait de réglisse.

**Revendications**

1. Enzyme purifiée d'Aspergillus terreus du type bêta-glucuronidase, d'un poids moléculaire de 95.000 daltons.

2. Enzyme bêta-glucuronidase selon la revendication 1, caractérisée en ce que, en phase aqueuse, elle possède un maximum d'activité entre 40 ° C et 50 ° C, de préférence 45 ° C et un pH entre 5 et 7, de préférence 6.

3. Enzyme selon l'une des revendications 1 et 2, caractérisée en ce qu'elle est fixée sur un support comme le nylon activé au glutaraldéhyde ou une bille acrylique activée à l'oxirane.

4. Procédé de production d'une enzyme du type bêta-glucuronidase selon la revendication 1 ou 2, comportant la culture d'un microorganisme, effectuée dans un milieu comportant au moins une source de carbone et une source d'azote, et l'extraction et la purification de l'enzyme à partir de la biomasse et/ou du milieu de culture, caractérisé en ce que l'enzyme est produite par la culture d'un microorganisme du type Aspergillus terreus ou de ses variants et mutants dans un milieu de culture comportant comme source de carbone une substance contenant un ou plusieurs glucuronides.

5. Procédé selon la revendication 4, caractérisé en ce que la production de l'enzyme est induite par la présence de glycyrrhizine dans le milieu de culture, comme source de carbone.

6. Procédé selon l'une des revendications 4 ou 5, caractérisé en ce que la culture d'Aspergillus terreus et effectuée dans un milieu contenant comme source d'azote des substances chosies parmi des sels d'ammonium, des sels de nitrate ou des acides aminés.

7. Procédé selon l'une des revendications 4 à 6, caractérisé en ce que le mono-ammonium de glycyrrhizinate est utilisé comme source de carbone et d'azote pour la culture d'Aspergillus terreus.

8. Procédé selon l'une des revendications 4 à 7, caractérisé en ce que la culture d'Aspergillus terreus est effectuée dans un milieu contenant outre une source de carbone et une source d'azote, des substances inorganiques choisies parmi des sels minéraux favorisant la croissance des moisissures.

9. Procédé selon l'une des revendications 4 à 8 caractérisé en ce que la culture d'Aspergillus terreus est effectuée dans un milieu contenant des sels minéraux choisis notamment parmi des sulfates de magnésium, sulfate de fer, sulfate de cuivre, sulfate de manganèse, chlorure de calcium, molybdate de sodium.

10. Procédé selon l'une des revendications 4 à 9, caractérisé en ce que la culture d'Aspergillus terreus est effectuée dans un milieu contenant en outre des vitamines favorisant la croissance des moisissures Aspergillus.

11. Procédé selon l'une des revendications 4 à 10, caractérisé en ce que la culture d'Aspergillus terreus est effectuée dans un milieu contenant des vitamines choisies notamment parmi : l'acide folique, la biotine, la choline, la nicotinamide, la panthothénate de calcium, le pyridoxale, la thiamine, la riboflavine.

12. Procédé selon l'une des revendications 4 à 11, caractérisé en ce que la température de culture est comprise entre 28 ° C et 45 ° C et de préférence 37 ° C et 44 ° C.

13. Procédé selon l'une des revendications 4 à 12 caractérisé en ce le pH du milieu de culture est compris entre 6 et 8.

14. Procédé selon l'une des revendications 4 à 13, caractérisé en ce que la culture est effectuée en milieu liquide ou solide, mais de préférence liquide.

**15.** Procédé d'hydrolyse d'une solution de glycyrrhizine ou d'extraits végétaux en contenant, et contenant ou non jusqu'à 20% en volume d'alcool éthylique, caractérisé en ce qu'on ajoute à la solution de la bêta-glucuronidase, selon l'une des revendications 1 à 3.

**16.** Procédé d'hydrolyse selon la revendication 15 caractérisé en ce qu'on opère à pH entre 4,5 et 6,5, de préférence 5,5.

**17.** Procédé selon les revendications 15 et 16, caractérisé en ce qu'on opère à température entre 35 et 55 °C, de préférence 40 °C.

**18.** Procédé de production d'acide 18 bêta-glycyrrhétinique, caractérisé en ce qu'on hydrolyse de la glycyrrhizine ou un extrait végétal en contenant, selon le procédé d'une des revendications 15 à 17.

**Claims**

**1.** A purified enzyme of the $\beta$-glucuronidase type from Aspergillus terreus of a molecular weight of 95.000 daltons.

**2.** The $\beta$-glucuronidase enzyme according to claim 1, which, in aqueous phase, has a maximum activity at between 40 °C and 50 °C, preferably 45 °C, and a pH of between 5 and 7, preferably 6.

**3.** The enzyme according to claim 1 or 2, which is fixed to a support such as nylon activated with glutaraldehyde or an acrylic bead activated with oxirane.

**4.** A process for the production of an enzyme of the $\beta$-glucuronidase type, according to claim 1 or 2, which comprises the culture of a microorganism, carried out in a medium containing at least one source of carbon and one source of nitrogen, and the extraction and purification of the enzyme from the biomass and/or culture medium, wherein the enzyme is produced by the culture of a microorganism of the Aspergillus terreus type, or variants and mutants thereof, in a culture medium in which a substance containing one or more glucuronides is present as a source of carbon.

**5.** The process according to claim 4, wherein the production of the enzyme is induced by the presence of glycyrrhizin as a source of carbon in the culture medium.

**6.** The process according to any one of the claims 4 or 5, wherein the culture of Aspergillus terreus is carried out in a medium containing, as a source of nitrogen, substances selected from ammonium salts, nitrate salts and amino acids.

**7.** The process according to any one of claims 4 to 6, wherein monoammonium glycyrrhizinate is used as a source of carbon and nitrogen for the culture of Aspergillus terreus.

**8.** The process according to any one of claims 4 to 7, wherein the culture of Aspergillus terreus is carried out in a medium containing, in addition to a source of carbon and a source of nitrogen, inorganic substances selected from mineral salts favoring mold growth.

**9.** The process according to any one of the claims 4 to 8, wherein the culture of Aspergillus terreus is carried out in a medium containing mineral salts selected in particular from magnesium sulfate, iron sulfate, copper sulfate, manganese sulfate, calcium chloride and sodium molybdate.

**10.** The process according to any one of the claims 4 to 9, wherein the culture of Aspergillus terreus is carried out in a medium also containing vitamins favoring the growth of the Aspergillus terreus molds.

**11.** The process according to any one of the claims 4 to 10, wherein the culture of Aspergillus terreus is carried out in a medium containing vitamins selected in particular from : folic acid, biotin, choline, nicotinamide, calcium panthothenate, pyridoxal, thiamine and riboflavin.

**12.** The process according to any one of the claims 4 to 11, wherein the culture temperature is between 28 °C and 45 °C, preferably between 37 °C and 44 °C.

13. The process according to any one of the claims 4 to 12, wherein the pH of the culture medium is between 6 and 8.

14. The process according to any one of claims 4 to 13, wherein the culture is carried out in a liquid or solid medium, but preferably a liquid medium.

15. A process for the hydrolysis of a solution of glycyrrhizin or plant extracts containing glycyrrhizin, which may or may not contain up to 20% by volume of ethyl alcohol, wherein β-glucuronidase according to any one of claims 1 to 3 is added to the solution.

16. The hydrolysis process according to the preceding claim, wherein the working pH is between 4.5 and 6.5, preferably 5.5.

17. The process according to claim 16 or claim 17, wherein the working temperature is between 35 and 55 ° C, preferably 40 ° C.

18. A process for the production of 18β-glycyrrhetinic acid, wherein glycyrrhizin or a plant extract containing glycyrrhizin is hydrolyzed by the process according to any one of claims 15 to 17.

**Patentansprüche**

1. Gereinigtes Enzym von Aspergillus terreus des Typs beta-Glucuronidase mit einem Molekulargewicht von 95.000 Dalton.

2. Beta-Glucuronidase-Enzym nach Anspruch 1, dadurch gekennzeichnet, daß es in wäßriger Phase ein Aktivitätsmaximum zwischen 40 ° C und 50 ° C, vorzugsweise von 45 ° C, und einen pH-Wert zwischen 5 und 7, vorzugsweise von 6, besitzt.

3. Enzym nach einem der Ansprüche 1 und 2, dadurch gekennzeichnet, daß es auf einem Träger wie Nylon, aktiviert mit Glutaraldehyd, oder auf einer Acrylkugel, aktiviert mit Oxiran, fixiert ist.

4. Verfahren zur Herstellung eines Enzyms des Typs beta-Glucuronidase nach Anspruch 1 oder 2, umfassend die Kultur eines Mikroorganismus, durchgeführt in einem Medium, das mindestens eine Kohlenstoffquelle und eine Stickstoffquelle aufweist, und die Extraktion und die Reinigung des Enzyms ausgehend von der Biomasse und/oder dem Kulturmedium, dadurch gekennzeichnet, daß das Enzym durch die Kultur eines Mikroorganismus vom Typ Aspergillus terreus oder seiner Varianten und Mutanten in einem Kulturmedium produziert wird, das als Kohlenstoffquelle eine Substanz umfaßt, die ein oder mehrere Glucuronide enthält.

5. Verfahren nach Anspruch 4, dadurch gekennzeichnet, daß die Produktion des Enzyms durch die Anwesenheit von Glycyrrhizin als Kohlenstoffquelle im Kulturmedium induziert wird.

6. Verfahren nach einem der Ansprüche 4 oder 5, dadurch gekennzeichnet, daß die Kultur von Aspergillus terreus in einem Medium durchgeführt wird, das als Stickstoffquelle Substanzen enthält, die unter Ammoniumsalzen, Nitratsalzen oder Aminosäuren ausgewählt werden.

7. Verfahren nach einem der Ansprüche 4 bis 6, dadurch gekennzeichnet, daß das Mono-Ammonium-Glycyrrhizinat als Kohlenstoffquelle und als Stickstoffquelle für die Kultur von Aspergillus terreus verwendet wird.

8. Verfahren nach einem der Ansprüche 4 bis 7, dadurch gekennzeichnet, daß die Kultur von Aspergillus terreus in einem Medium durchgeführt wird, das außer einer Kohlenstoffquelle und einer Stickstoffquelle anorganische Substanzen umfaßt, ausgewählt unter den Mineralsalzen, die das Wachstum des Schimmels begünstigen.

9. Verfahren nach einem der Ansprüche 4 bis 8, dadurch gekennzeichnet, daß die Kultur von Aspergillus terreus in einem Medium durchgeführt wird, das Mineralsalze enthält, insbesondere ausgewählt unter den Magnesiumsulfaten, Eisensulfat, Kupfersulfat, Mangansulfat, Calciumchlorid und Natriummolybdat.

**10.** Verfahren nach einem der Ansprüche 4 bis 9, dadurch gekennzeichnet, daß die Kultur von Aspergillus terreus in einem Medium durchgeführt wird, das außerdem Vitamine enthält, die das Wachstum des Schimmels von Aspergillus begünstigen.

**11.** Verfahren nach einem der Ansprüche 4 bis 10, dadurch gekennzeichnet, daß die Kultur von Aspergillus terreus in einem Medium durchgeführt wird, das Vitamine enthält, insbesondere ausgewählt unter Folsäure, Biotin, Cholin, Nikotinamid. Calciumpanthotenat, Pyridoxal, Thiamin und Riboflavin.

**12.** Verfahren nach einem der Ansprüche 4 bis 11, dadurch gekennzeichnet, daß die Temperatur der Kultur zwischen 28 °C und 45 °C und vorzugsweise zwischen 37 °C und 44 °C liegt.

**13.** Verfahren nach einem der Ansprüche 4 bis 12, dadurch gekennzeichnet, daß der pH-Wert des Kulturmediums zwischen 6 und 8 liegt.

**14.** Verfahren nach einem der Ansprüche 4 bis 13, dadurch gekennzeichnet, daß die Kultur im flüssigen oder festen Medium durchgeführt wird, jedoch vorzugsweise im flüssigen.

**15.** Verfahren zur Hydrolyse einer Lösung von Glycyrrhizin oder von Pflanzenextrakten, die dieses enthalten, und die eventuell bis zu 20 Vol.-% Ethylalkohol enthalten, dadurch gekennzeichnet, daß man zu der Lösung beta-Glucuronidase nach einem der Ansprüche 1 bis 3 gibt.

**16.** Verfahren zur Hydrolyse nach Anspruch 15, dadurch gekennzeichnet, daß man bei einem pH-Wert zwischen 4,5 und 6,5, vorzugsweise bei 5.5, arbeitet.

**17.** Verfahren nach Anspruch 15 und 16, dadurch gekennzeichnet, daß man bei einer Temperatur zwischen 35 °C und 55 °C, vorzugsweise bei 40 °C, arbeitet.

**18.** Verfahren zur Herstellung von 18-$\beta$-Glycyrrhetinsäure, dadurch gekennzeichnet, daß man Glycyrrhizin oder einen Pflanzenextrakt, der dieses enthält gemäß dem Verfahren nach einem der Ansprüche 15 bis 17 hydrolysiert.